# EUROPEAN PATENT SPECIFICATION

(11) **EP 0 952 785 B1**
(45) Date of publication and mention of the grant of the patent: **02.01.2004**
(21) Application number: 97945481.6
(22) Date of filing: 01.10.1997
(51) Int. Cl.: G01N 27/02, A61B 5/113

(54) **CONDUCTANCE MEASURING APPARATUS**
KONDUKTANZMESSAPPARAT
APPAREIL DE MESURE DE LA CONDUCTANCE

(30) Priority: 01.10.1996 US 724388
(43) Date of publication of application: 03.11.1999
(73) Proprietor: Intellectual Property LLC, Cross Plains, Wisconsin 53528 (US)
(72) Inventor: REINING, William, N., Cross Plains, WI 53528 (US); BELL, Marshall, J., Stoughton, WI 53589 (US)
(74) Representative: Calderbank, Thomas Roger
(86) International application number: PCT/US1997/017897
(87) International publication number: WO 1999/007281

(56) References cited:
- DE-A- 2 210 296
- DE-A- 3 532 520
- GB-A- 2 012 431
- GB-A- 2 116 725
- US-A- 4 258 718
- US-A- 4 279 257
- US-A- 5 131 399
- US-A- 5 291 782

## Description

### Field of the Invention

The present invention relates to a sensor for measuring conductance of material and in particular a sensor that may be used, among other applications, for measuring periodic respiratory and cardiac motion by detecting changes in the conductance of patient tissue.

### Background of the Invention

### Conductance Measurement

The conductance of a material, that is, how well it conducts electricity, may be determined by applying a voltage to the material with a pair of electrodes and measuring the corresponding current flow.

This approach, while straightforward, has several disadvantages. First, it is sensitive to localized high resistances at the interface between the electrodes and the material. Second, the electrodes used to apply the voltage must contact the material raising issues of contamination of the materials and corrosion of the electrodes.

For this reason, it is known to make non-contacting measurement of conductivity using a pair of electrical coils immersed in the material being measured. The premise behind such systems is that electrical coupling of an alternating current signal between the two coils is functionally related to the conductivity of the material between the coils. Such systems have the disadvantage of requiring multiple coils.

A single coil resistivity measuring system (resistivity being the inverse of conductivity) is described in U.S. Patent 4,536,713. In this system, eddy currents are induced in a drilling fluid for an oil well or the like by a magnetic field applied via a ferrite core. The measured eddy currents, as reflected on the driving voltage of the coil, provide a coarse indication of drilling liquid resistivity.

### Respiration Monitoring

Electronic monitoring of respiration may be performed for critically ill or comatose patients, small children at risk of sudden infant death ("SID"), or patients with sleep apneas (partial suspension of breathing sometimes associated with insomnia).

There are two types of sleep apnea. Obstructive apnea, is caused by blockage of the windpipe or mouth and nose, and is characterized by a change in the phase relationship between respiratory movement of the chest and abdomen. In central apnea, the patient ceases all signs of breathing.

Distinguishing central apnea from obstructive apnea requires independent detection of abdominal and thoracic respiratory motion.

A number of different types of respiration sensors are known in the art, including mechanical sensors employing air flow measuring turbines or the like, that require the use of a face mask or mouthpiece, and inflatable cuffs which measure chest or abdomen extension manifest as a change in cuff pressure. Such mechanical systems can suffer from problems of reliability.

Respiratory motion may be also detected electronically by conductive coils wrapped around the patient. In one prior art device, multiple inductive coils are positioned on the patient to move with respect to each other as the patient breathes. The mutual inductance between the coils changes with their relative motion and is measured to detect respiratory motion.

In a somewhat different design, respiratory deformation of a single coil causes a change of inductance in that coil which may be measured by the detuning of an oscillator using the coil's inductance as a tuning element. The change in frequency of the oscillator is used as a measure of respiratory motion.

With these electrical sensors, the sensing coil must be snugly fit to the patient so as to move with the patient and for this reason may create an unpleasant confining sensation or pressure on the patient's trunk and stomach. Such constraining pressure is particularly problematic with small infants. Such electronic sensors are unsuitable for measuring cardiac motion which does not result in significant distention of the patient's chest or abdomen.

US-A-4536713 mentioned above discloses a sensor for measuring the conductivity of a flowing drilling fluid in which a coil providing an inductor and a capacitor are connected in series to form a resonant circuit. That circuit is driven by an oscillator to cause the coil to produce an oscillating magnetic filed which induces eddy currents in the fluid. A differential amplifier then measures the effective resistance of the resonance circuit, to derive a measurement of the conductivity of the fluid.

According to the present invention there is provided
a conductivity sensor for measuring a proximate material comprising:
a conductive coil providing an inductor producing an oscillating magnetic field inducing eddy currents in the proximate material when the conductive coil is energized with an oscillating current;
a capacitor connected in parallel with the inductor to produce a parallel resonant circuit having a resonant frequency; and
an oscillator connected across the parallel resonant circuit to drive the parallel resonant circuit with the oscillating current; and
an impedance measuring circuit arranged to provide a measure of an effective resistance of the parallel resonance circuit;
wherein
the oscillator is an auto-tuning oscillator including an amplifier having an output and input connected to the parallel resonant circuit to provide positive feedback causing the oscillating current from the output to be at a resonant frequency of the parallel resonance circuit as coupled to the proximate material to induce eddy currents in the proximate material; and
the impedance measuring circuit is arranged to control the oscillating current according to an error signal to produce a constant amplitude alternating voltage across the inductor, the error signal being the difference between a standard reference signal and a measure of the amplitude of the voltage across the inductor whereby the impedance measuring circuit provides a measure of the effective resistance of the parallel resonant circuit as coupled to the proximate material, as reflects the magnitude of the induced eddy currents within the proximate material, according to the amplitude of the error signal.

Thus, the invention may enable a simplified conductance measuring sensor to be produced, which is resistant to inductive and capacitive effects. By driving the coil at its natural resonant frequency, the resistive effects to be measured dominate.

The auto-tuning oscillator may include a gain controlled amplifier having a gain control input signal reflecting the amplitude of the signal driving the coil, and the impedance measuring circuit, may derive resistance from the gain control signal.

Thus it is another object of the invention to provide a conductance sensor that is resistant to amplitude dependent effects in the conductance measuring process.

The gain control signal may be provided by a synchronous rectifier, rectifying an oscillator signal from the oscillating current synchronously with the oscillating current. The synchronous rectifier may be a multiplier multiplying the oscillator signal by itself.

Thus it is another object of the invention to provide a conductance sensor that is resistant to asynchronous noise sources. The synchronous rectifier is not switched by larger amplitude out-of-phase or different frequency signals which will then be averaged out in a subsequent filtering stage.

One application of the conductance sensor is electronic monitoring of respiratory (or cardiac) motion which relies on the measurement of changes in conductivity in the patient's tissue within a limited cross-sectional area. Because the measurement does not rely on changes in the coil's dimensions, the sensor may be less constraining to the patient and more robust against deformation of the coil in use.

Specifically, the conductive coil is sized to fit about the body of a person. The auto-tuning oscillator, connected to the conductive coil, drives the coil with an oscillating current at a resonant frequency to induce eddy currents in the person. Attached to the conductive coil is the impedance measuring circuit which provides a measure of the effective resistance of the coil at the first frequency as reflects the magnitude of the induced eddy currents within the person. An electronic filter receiving this impedance signal extracts a periodic plethysmographic signal.

Thus, it is one object of the invention to provide a method of measuring periodic physiological motion, such as cardiac or respiratory motion, by detecting changes in areal conductance of patient tissue within a coil without the need to deform the coil in time with the physiological motion. Because the invention does not require outward distention of the patient, it is equally effective to measure periodic internal conductance changes, for example those caused by the heartbeat, as with those conductance changes which are reflected in outward movement of the patient, such as breathing.

The patient monitor may include two coils sized to fit about the body of the person, one in the abdominal area of the person and one in the thoracic area of the patient. Here, the impedance measuring circuit provides measures of the effective resistance of the two coils separately. An alarm may compare these separate resistance measurements to determine when distress of the person is indicated.

Thus, it is another object of the invention to provide an electronic respiratory detector that can provide separate measures of two different regions of the body in proximity to each other. Because only a single coil is needed to measure conductivity, multiple measurements may be made by adjacent multiple coils.

Preferably, a conductive shield surrounds the coil or the side of the coil away from the patient.

There may also be a conductive outer sleeve fitted coaxially around the outside of the first and second coils and the inner sleeve.

The foregoing and other objects and advantages of the invention will appear from the following description. In the description, reference is made to the accompanying drawings which form a part hereof and in which there is shown by way of illustration, a preferred embodiment of the invention. Such embodiment does not necessarily represent the full scope of the invention, however, and reference must be made therefore to the claims herein for interpreting the scope of the invention.

### Brief Description of the Drawings

Figure 1 is a perspective view of a belt incorporating the present invention and shown in position on an infant;
Figure 2 is a perspective view of the belt of Figure 1 when open showing the localization of the magnetic fields from two coils in the abdominal and thoracic region;
Figure 3 is a plot of magnetic field strength versus distance along line 3-3 of Figure 2 when the belt is closed;
Figure 4 is a schematic diagram of a circuit used to drive the coils of Figure 2 and to provide a conductance measurement of the region of the magnetic fields shown in Figure 2;
Figure 5 is a block diagram showing subsequent processing of the conductance measurement; and
Figure 6 is a perspective view of a general-purpose conductance sensor employing the principle of the present invention.

### DETAILED DESCRIPTION OF THE PREFERRED EMBODIMENT

### Belt Construction and Loop Placement.

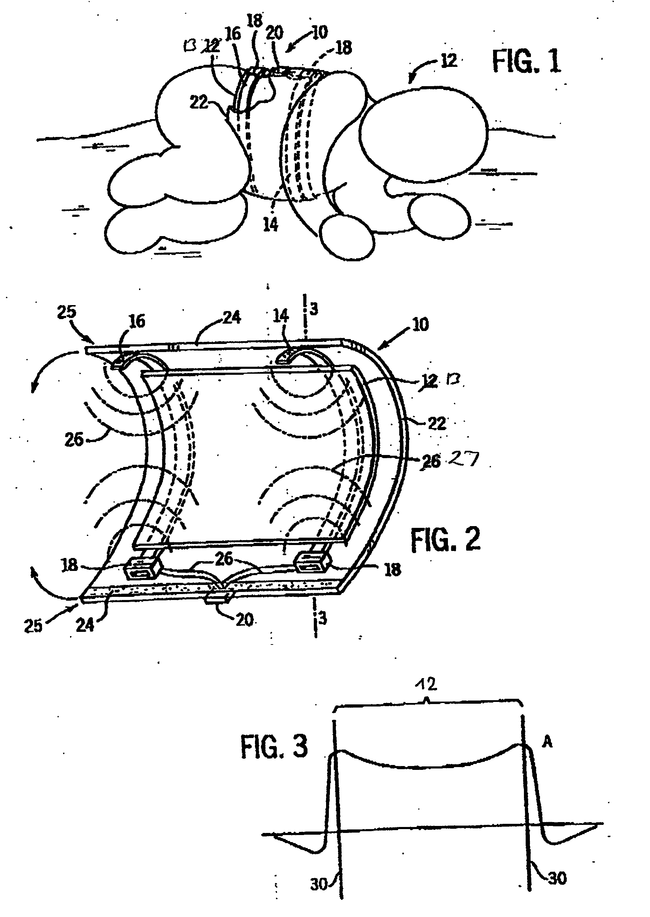

Referring to Figure 1, the present invention may be incorporated into a belt 10 for wrapping about the trunk of a patient 12, such as an infant, with the upper edge of the belt being positioned about the patient's thoracic region and the lower edge of the belt being positioned about the patient's abdomen.

Referring also to Figure 2, the belt 10 may include an inner fabric sleeve 13 providing electrical insulation and cushioning to the patient 12 against various rigid portions of the belt 10 as will be described.

Attached to the outside of the sleeve 13 near the superior and inferior edges of the belt 10 are first and second flexible electrical conductors 14 and 16 which open into a C-shaped form when the belt 10 is opened. Connectors 18, attached to one end of each of conductors 14 and 16, physically join together the ends of conductors 14 and 16, to form two loops, one about the thoracic and one about the abdominal regions of the patient 12 when the belt 10 is closed, as shown in Figure 1. The opposing ends of each of the conductors 14 and 16 remain electrically isolated so that an oscillating electrical signal may be applied across the ends of conductor 14 and conductor 16.

The conductors 14 and 16 may be flat metal springs of a conductive material such as brass so as to open and close without permanent deformation.

An outer insulating fabric sleeve 22 covers the inner sleeve 12, sandwiching the conductors 14 and 16 between the inner sleeve 13 and the outer sleeve 22. Medial edges 25 of the outer sleeve 22 support opposite halves of a strip fastener 24, such as velcro, so that the medial edges 25 may be removably attached to one another to close the belt 10 about the patient 12 and to resist outward forces which might serve to disconnect conductors 14 and 16 from connectors 18.

Additional circuitry 20 which includes two oscillators, to be described below, and a low powered radio transmitter to transmit signals from the patient 12, and a battery is connected to each of the connectors 18 and thus to both ends of each conductor 14 and 16 by wires 26 running along the inner surface of the outer sleeve 22 and passing through the outer sleeve 22 to the circuitry 20.

### Conductivity Measurement with Eddy Currents

Referring now also to Figure 3, when the conductors 14 and 16 are formed into a loop with a closing of the belt about the patient 12, alternating current passing through the conductors 14 and 16 generates oscillating magnetic fields 26 within the patient 12. Figure 3 shows the amplitude of magnetic fields along a transverse plane through the patient caused by current flow through conductors 14 and 16.

As shown in Figure 3, the amplitude of the magnetic field A is concentrated within the patient 12 and of relatively low magnitude outside of the patient, the boundaries of which are indicated by vertical lines 30. The strength of the magnetic field falls off rapidly in a direction perpendicular to the plane of the coils of the conductors 14 and 16 and thus interference in the measurements obtained by the coils 14 and 16 is low.

The oscillating magnetic fields 27 will induce eddy currents within the patient 12, the magnitude of which are proportional to the conductivity of the tissue. For example, if the tissue of the patient 12 were perfectly conductive, the eddy currents would be sufficient to generate a magnetic field (back EMF) opposing the magnetic field 27 to perfectly cancel that magnetic field 27. On the other hand, to the extent that the tissue of the patient 12 is not perfectly conductive, the eddy fields will be lower, and the magnetic field 26 will be reduced but not canceled. Thus, a measurement of the back EMF creared by the eddy currents within the patient provides an indication of the conductivity of the patient tissue.

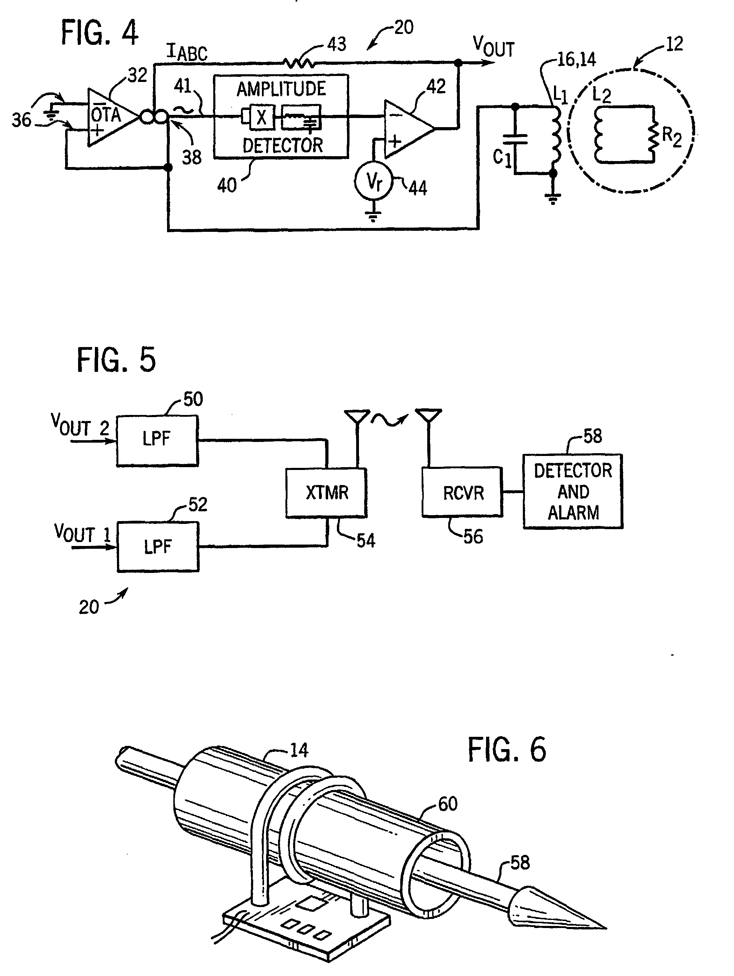

Referring now to Figure 4, the effective circuit of a conductor 14 or 16 in proximity to the patient 12 may be modeled as a first inductor L1 (comprised of either conductor 14 or conductor 16), inductively coupled (as if in a transformer) to a second inductor L2, connected in series with a resistance R₂. The inductor L2 and resistance R2 are not discrete elements, but are the equivalent lumped values incorporating the distributed inductance and resistance of many looping current paths of the eddy currents through tissue of the patient 12. Generally, however, R₂ will reflect a total conductivity (1/R) in the region within a given loop 14 and 16.

### Relationship Between Conductivity And Physiological Motion

This resistance R₂ in turn will depend generally on the real resistance (Ω), of the tissue in the area enclosed by the loop 14 or 16 times the area enclosed by the loop 14 or 16. For this reason, changes in the cross-sectional area of the patient 12 within either loop 14 or 16 will produce changes in the value of R₂ measured with that loop 14 or 16.

Further, changes in the cross-sectional area of an organ within the patient 12 and within the plane of a loop 14 or 16, and having a noticeably different areal resistance than the surrounding tissue will also be reflected in a change in the value of R₂. For this reason, the value of R₂ will show both respiratory motion in which the patient's overall cross-sectional area changes, and cardiac motion in which a relative area of highly conductive blood changes within the patient without significant overall change in the overall patient cross-sectional area.

### Resistance Measuring Circuitry

The value of R₂ is detectable as a decrease in the quality factor of an oscillator employing conductor 14 or 16 as an inductive component of an oscillator tank circuit. The oscillator is formed from the parallel combination of inductance L1 being one of conductor 16 or 14, and a capacitor C₁ contained within the electronics circuitry 20. In a preferred embodiment, C₁ is selected to tune the combination of L1 and C1 into parallel resonance at approximately 4.5 MHz. For conductors 14 and 16 formed into a loop having a diameter of approximately 100 millimeters, C₁ will be a capacitor of 330 pF.

As is known in the art, the quality factor of an oscillator provides a measure generally indicating how long an oscillator would continue to oscillate without the input of additional energy (free oscillation). Without eddy currents, the oscillator formed of conductors 14 or 16 would be expected to oscillate for a time limited only by the intrinsic resistance of the conductors 14 and 16 and the other components forming the oscillating resonant circuit. With eddy currents, the resulting back EMF adds an effective power dissipating resistance to the oscillator, shortening the time of free oscillation. Thus, a measure of the quality factor of oscillators formed from conductors 14 and 16 provides an indication of the conductivity of the patient 12.

The measurement the of quality factor of a resonant circuit is well known in the art. However, in the present invention, an extremely precise measurement is required and the measurement must be robust in the environment of a moving patient and in a situation where the value of L₁ may change from measurement-to-measurement based on some inadvertent deformation of the conductors 14 and 16, and in variations of attaching the belt 10 to the patient 12.

In order to improve the quality factor measurement, the measurement is made at the resonant frequency of the parallel combination of C₁ and L₁. In fact, the oscillator driving the loops 14 and 16 effectively tracks to the resonant frequency of the parallel combination of C₁ and L₁ as will be described. Thus the effect of variations in the resonant frequency of L₁ and C₁ with respect to the oscillator frequency, such as might effect the quality factor measurement, is reduced. It will be further understood that by driving the coil at resonant frequency, undesired capacitive and inductive influences on the measurement are reduced because the inductive components of the detected signal will cancel the capacitive components of that signal.

In addition, the amplitude of the oscillator driving the parallel combination of L₁ and C₁ is precisely controlled to a constant value. Thus, the effect of amplitude on the quality factor measurement is eliminated and apparent changes in quality factors such as might be caused by a slight detuning of the oscillator with respect to the inductive parallel combination of L1 and C1, are reduced.

In a preferred embodiment, an oscillator providing the desired feature of tracking the resonance of C₁ and L₁ and providing a constant amplitude oscillation is provided through the use of an operational transconductance amplifier (OTA) 32 connected at its output 38 to one junction between capacitor C₁ and inductor L₁. The remaining junction between C₁ and L₁ is connected to ground.

As is understood in the art, an operational transconductance amplifier provides a current at its output 38 that may be modeled as a gain factor Gₘ times the voltage across inverting and non-inverting inputs 36 (indicated by a minus and plus sign, respectively) of the operational transconductance amplifier 32. The value Gₘ is determined by an amplifier bias current I_{abc}·

The output 38 of the OTA 32 is also connected to the non-inverting input 36 of the OTA 32. In this positive feedback configuration, the output current of the OTA 32 will naturally oscillate at the resonant frequency of the parallel combination of C₁ and L₁ to produce an oscillator signal 41.

This oscillator signal 41 on output 38 is also received by an amplitude detector 40 which is a precision synchronous rectifier and low-pass filter providing a DC voltage proportional to the amplitude of the oscillator signal 41 at output 38. The synchronous rectifier is realized in the preferred embodiment by a multiplier that accepts at its two factor inputs the output 38. Any noise signal on output 38 that is asynchronous with the oscillator signal 41 will average to zero in the low pass filter. The output of the amplitude detector 42 is received by an inverting input of a standard high-gain operational amplifier 42, whose positive input receives a precision reference voltage 44 designated as Vᵣ.

The amplifier 42 operates open-loop, and hence it will be understood that if the voltage on the inverting input of the amplifier 42 is greater than Vᵣ, the output of amplifier 42 will be a negative value. On the other hand, if the voltage on the inverting input of the amplifier 42 is negative with respect to Vᵣ, the output of amplifier 42 will be positive. The output of the amplifier 42, termed Vₒᵤₜ, is applied to limiting resistor 43 to become amplifier bias current I_{abc}.

The connection of the output of the amplifier 40 to the OTA 32 provides feedback control of the amplitude of the oscillator signal 41 to the value of Vᵣ. As connected in this manner, the value of Vₒᵤₜ being the amplitude error signal indicates generally how much additional energy must be input into the oscillator of C₁ and L₁ to maintain oscillation at the desired amplitude of Vᵣ and thus provides a measure of the quality factor of the oscillator of C₁ and L₁ and hence a measure of R₂. Generally, the higher the value of Vₒᵤₜ, the less the value of R₂.

Referring now to Figure 5, the circuit of Figure 4 is repeated once for each of the conductors 14 and 16 and hence two values of Vₒᵤₜ will be obtained, each indicating the conductivity of the patient within the area encircled by respective loops 14 and 16. These two signals distinguished as Vₒᵤₜ₂ (referring to the signal from coil 16 and from the abdomen) and Vₒᵤₜ₁ (referring to the signal from coil 14 and from the thoracic region), are each received by separate low-pass filters 50 and 52, respectively, which are tuned to reject the fundamental frequency of the oscillator signal 42 driving the loops 14 and 16, but to pass lower frequency signals caused by a slowly varying change in the value of R₂ with respiration or cardiac motion. In the following discussion, it will be assumed that the frequency of the low-pass filters 50 and 52 is adjusted to extract respiratory motion such as may be needed to diagnose sleep apnea.

The signals from the low-pass filters 50 and 52 are then provided to a low-powered radio transmitter 54 contained within the circuitry 20 shown with respect to Figure 2. A nearby receiver 56 receiving this signal extracts two respiratory signals. The transmitter and receiver circuits 54 and 56 are constructed according to techniques well-known in the art, and may be a simple low-power FM link with a different frequency assigned to each of the different respiratory signals.

The receiver 54 provides the respiratory signals to a detector and alarm circuit 58 which is programmed to detect patient distress. The detector and alarm circuit 58 may provide an alarm indicating a loss of respiratory signal extending for more than a certain period of time. Alternatively, or in addition, the alarm and detector circuit 58 may detect a loss of phase coherence between the signals Vₒᵤₜ₁ and Vₒᵤₜ₂ such as may indicate obstructive apnea as described before. The detector and alarm 58, in a preferred embodiment, are a microprocessor programmed to receive digitized samples of the respiratory signals and to monitor them according to the criteria described above or other criteria that may be selected by a physician.

It will be recognized that this approach of detecting cardiac and respiratory signals through a measurement of changes in conductivity does not require that the loops 14 and 16 be deformed or flexed with motion of the patient 12, and in fact, the circuit would work if loops 14 and 16 were perfectly rigid and unyielding. As a result, the patient 12 is not unduly compressed about the torso by pressure by the belt 10 other than that minimally necessary to hold the belt 10 in position.

Referring now to Figure 6, the above-described circuit may also find use in measuring the conductivity of an arbitrary material, such as a liquid 58 passing through a pipe 60. Here a single conductor 14 is used passing in one or more loops about the circumference of the tube 60 so as to again generate an internal oscillating magnetic field (not shown) inside the tube 60. The back EMF of the eddy currents so induced is detected by measuring the quality factor of an oscillator formed of the conductor and a parallel capacitor (not shown) as described above.

Unlike other conductance sensors in the prior art, no direct contact is required between the liquid 58 and one or more electrodes of the circuit, nor is it necessary that the loop 14 be immersed in the liquid 58 such as may produce clogging or unnecessary turbulence, or that the loop 14 be used in conjunction with a second loop 16.

The measurement of conductivity in this manner may provide an indication of the composition of the liquid 58 and may detect inclusions such as air bubbles or the like.

The liquid 58 need not fill the tube 60, however. To the extent that the liquid 58 occupies only a portion of the cross-sectional area excited by the coil 14, the conductivity will look proportionately lower.

Many other modifications and variations of the preferred embodiment which will still be within scope of the invention as defined in the claims will be apparent to those with ordinary skill in the art. For example, the conductance probe may be used over a large volume and thus as a means of detecting the presence of people in a room. On a smaller scale the conductance sensor may be used to detect nearby high or low conductance materials including metal and thus function as a proximity switch. In order to apprise the public of the various embodiments that may fall within the scope of the invention, the following claims are made.

## Claims

1. A conductivity sensor for measuring a proximate material comprising:
a conductive coil (14, 16) providing an inductor (L1) producing an oscillating magnetic field inducing eddy currents in the proximate material when the conductive coil is energized with an oscillating current;
a capacitor (C₁) connected in parallel with the inductor to produce a parallel resonant circuit having a resonant frequency; and
an oscillator (32) connected across the parallel resonant circuit to drive the parallel resonant circuit with the oscillating current; and
an impedance measuring circuit (40, 42) arranged to provide a measure of an effective resistance of the parallel resonance circuit;
wherein the oscillator (32) is an auto-tuning oscillator including an amplifier having an output and input connected to the parallel resonant circuit to provide positive feedback causing the oscillating current from the output to be at a resonant frequency of the parallel resonance circuit as coupled to the proximate material to induce eddy currents in the proximate material; and
the impedance measuring circuit (40, 42) is arranged to control the oscillating current according to an error signal to produce a constant amplitude alternating voltage across the inductor, the error signal being the difference between a standard reference signal (44) and a measure of the amplitude of the voltage across the inductor (11) whereby the impedance measuring circuit provides a measure of the effective resistance of the parallel resonant circuit as coupled to the proximate material, as reflects the magnitude of the induced eddy currents within the proximate material, according to the amplitude of the error signal.

2. The conductivity sensor of claim 1 wherein the auto-tuning oscillator includes a gain controlled amplifier (32) having a gain control input signal reflecting the amplitude of the signal driving the coil; and
wherein the impedance measuring circuit derives the effective resistance from the gain control signal.

3. The conductivity sensor of claim 2 wherein the gain control signal is provided by a synchronous rectifier, rectifying an oscillator signal from the auto-tuning oscillator synchronously with the oscillating current.

4. The conductivity sensor of claim 2 wherein the gain control signal is provided by a rectifier in form of a multiplier multiplying the oscillating signal from the auto-tuning oscillator by itself.

5. The conductivity sensor of claim 1 including a guide (60) for positioning the measured material in a predetermined relationship with respect to the coil.

6. The conductivity sensor of claim 5 wherein the guide (60) is a non-conductive tube through which a liquid (58) may pass.

7. The conductivity sensor of claim 1 wherein the measured material is proximate but outside the coil (14, 16).

8. A patient monitor having a conductivity sensor according to any one of the preceding claims, wherein
the conductive coil (14, 16) is sized to fit about the body of a patient (12);
the auto-tuning oscillator (32) is arranged to induce eddy currents in the tissue of the patient;
the impedance measuring circuit (40, 42) is arranged to provide a measure of the effective resistance of the parallel resonant circuit as reflects the magnitude of the induced eddy currents within the tissue of the patient (12); and
the monitor has an electronic filter (50, 52) receiving the measure of impedance to extract a periodic plethysmographic signal.

9. The patient monitor of claim 8 wherein the filter (50, 52) is arranged to provide a periodic plethysmographic signal indicating respiration by the patient (12).

10. The patient monitor of claim 8 wherein the filter (50, 52) is arranged to provide a periodic plethysmograpic signal indicating the patient's heartbeat.

11. The patient monitor of claim 8 including in addition, a conductive shield surrounding the coil on the side of the coil away from the patient.

12. The patient monitor of claim 8 including an alarm circuit (58) arranged to monitor the periodic plethysmographic signal to produce an alarm signal when distress of the patient (12) is indicated.

13. The patient monitor of claim 12 having two of said conductive coils (14, 16) sized to fit about the body of the patient (12), one in an abdominal area of the patient (12) and one in a thoracic area of the patient wherein the impedance measuring circuit provides separate measures of the effective resistance of the parallel resonant circuits including the respective two coils (14, 16); and
wherein the alarm circuit (58) compares these separate resistance measures to determine when distress of the patient (12) is indicated.

14. A patient monitoring belt having a conductivity sensor according to any one of claims 1 to 8, wherein the belt has a fabric inner sleeve (13) sized to fit about the body of a patient (12);
said conductive coil comprises a first and a second coil (14, 16) positioned circumferentially around the sleeve in spaced apart relationship to fit one in an abdominal region and the other in a thoracic region of the patient when the sleeve (13) is in place on the patient (12);
said auto-tuning oscillator (32) comprises a first and a second auto-tuning oscillator for exciting the first and second coils into resonance; and
said impedance measuring circuit (40, 42) is arranged to provide a measure of the parallel resonant circuits respectively including the first and second coils (14, 16) at resonance.

15. The patient monitoring belt of claim 14 including a conductive outer sleeve fitting coaxially around the outside of the first and second coils (14, 16) and the inner sleeve (13).

## Patentansprüche

1. Leitfähigkeitssensor zum Messen eines in der Nähe befindlichen Materials, umfassend:
eine leitende Spule (14, 16), die eine Induktionsspule (L1) bereitstellt, die ein oszillierendes Magnetfeld erzeugt, das Wirbelströme im in der Nähe befindlichen Material induziert, wenn die leitende Spule mit einem oszillierenden Strom beaufschlagt wird;
einen Kondensator (C₁), der zu der Induktionsspule parallel geschaltet ist, um einen Parallel-Resonanzkreis bereitzustellen, der eine Resonanzfrequenz aufweist; und
einen Oszillator (32), der an den Parallel-Resonanzkreis geschaltet ist, um den Parallel-Resonanzkreis mit dem oszillierenden Strom anzusteuern; sowie
eine Impedanzmessschaltung (40, 42), die so angeordnet ist, dass sie einen Messwert eines Wirkwiderstands des Parallel-Resonanzkreises liefert;
worin der Oszillator (32) ein sich selbst abstimmender Oszillator ist, der einen Verstärker mit einem Ausgang und einem Eingang umfasst, der mit dem Parallel-Resonanzkreis verbunden ist, um eine positive Rückkopplung bereitzustellen, die bewirkt, dass sich der oszillierende Strom vom Ausgang auf einer Resonanzfrequenz des Parallel-Resonanzkreises befindet, der an das in der Nähe befindliche Material gekoppelt ist, um Wirbelströme im in der Nähe befindlichen Material hervorzurufen; und
die Impedanzmessschaltung (40, 42) so angeordnet ist, dass sie den oszillierenden Strom gemäß einem Fehlersignal steuert, um eine Wechselspannung mit konstanter Amplitude an der Induktionsspule zu erzeugen, wobei das Fehlersignal die Differenz zwischen einem Standardreferenzsignal (44) und einem Messwert der Amplitude der Spannung an der Induktionsspule (11) ist, wodurch die Impedanzmessschaltung einen Messwert des Wirkwiderstands des Parallel-Resonanzkreises, der an das in der Nähe befindliche Material gekoppelt ist, wie er der Größe der herbeigeführten Wirbelströme innerhalb des in der Nähe befindlichen Materials entspricht, entsprechend der Amplitude des Fehlersignals liefert.

2. Leitfähigkeitssensor nach Anspruch 1, worin der sich selbst abstimmende Oszillator einen verstärkungsgeregelten Verstärker (32) umfasst, der ein Verstärkungssteuereingangssignal aufweist, das der Amplitude des die Spule steuernden Signals entspricht; und
worin die Impedanzmessschaltung den Wirkwiderstand vom Verstärkungssteuersignal ableitet.

3. Leitfähigkeitssensor nach Anspruch 2, worin das Verstärkungssteuersignal durch einen synchronen Gleichrichter bereitgestellt wird, der ein Oszillatorsignal vom sich selbst abstimmenden Oszillator synchron mit dem oszillierenden Strom gleichrichtet.

4. Leitfähigkeitssensor nach Anspruch 2, worin das Verstärkungssteuersignal durch einen Gleichrichter in Form eines Vervielfachers bereitgestellt wird, der das oszillierende Signal vom sich selbst abstimmenden Oszillator selbst vervielfacht.

5. Leitfähigkeitssensor nach Anspruch 1, das eine Führung (60) umfasst, um das gemessene Material in einer vorbestimmten Beziehung in Bezug auf die Spule anzuordnen.

6. Leitfähigkeitssensor nach Anspruch 5, worin die Führung (60) eine nicht leitende Röhre ist, durch die eine Flüssigkeit (58) hindurchtreten kann.

7. Leitfähigkeitssensor nach Anspruch 1, worin sich das gemessene Material in der Nähe aber außerhalb der Spule (14, 16) befindet.

8. Patienten-Überwachungsgerät, das einen Leitfähigkeitssensor nach einem der vorangegangenen Ansprüche aufweist, worin
die leitende Spule (14, 16) eine solche Größe aufweist, dass sie um den Körper eines Patienten (12) passt;
der sich selbst abstimmende Oszillator (32) dazu angeordnet ist, Wirbelströme im Gewebe des Patienten hervorzurufen;
die Impedanzmessschaltung (40, 42) dazu angeordnet ist, einen Messwert des Wirkwiderstands des Parallel-Resonanzkreises zu liefern, wie er der Größe der herbeigeführten Wirbelströme im Gewebe des Patienten (12) entspricht; und
das Überwachungsgerät ein elektronisches Filter (50, 52) aufweist, das den Messwert der Impedanz empfängt, um ein periodisches plethysmographisches Signal zu extrahieren.

9. Patienten-Überwachungsgerät nach Anspruch 8, worin das Filter (50, 52) dazu angeordnet ist, ein periodisches plethysmograpisches Signal zu liefern, das die Atmung des Patienten (12) anzeigt.

10. Patienten-Überwachungsgerät nach Anspruch 8, worin das Filter (50, 52) dazu angeordnet ist, ein periodisches plethysmograpisches Signal zu liefern, das den Herzschlag des Patienten anzeigt.

11. Patienten-Überwachungsgerät nach Anspruch 8, das zusätzlich eine leitende Abschirmung umfasst, die die Spule auf der vom Patienten abgewandten Seite der Spule umgibt.

12. Patienten-Überwachungsgerät nach Anspruch 8, die eine Alarmschaltung (58) umfasst, die dazu angeordnet ist, das periodische plethysmographische Signal zu
überwachen, um ein Alarmsignal zu erzeugen, wenn Gefahr für den Patienten (12) angezeigt ist.

13. Patienten-Überwachungsgerät nach Anspruch 12, das zwei der leitenden Spulen (14, 16) in einer solchen Größe aufweist, dass sie um den Körper des Patienten (12) passen, wobei sich eine in einem Bauchbereich des Patienten (12) und eine im Brustbereich des Patienten befindet, worin die Impedanzmessschaltung getrennte Messwerte des Wirkwiderstands der Parallel-Resonanzkreise liefert, die die jeweiligen beiden Spulen (14, 16) umfassen; und
worin die Alarmschaltung (58) diese getrennten Widerstandsmesswerte vergleicht, um zu bestimmen, wann Gefahr für den Patienten (12) angezeigt ist.

14. Patienten-Überwachungsgürtel mit einem Leitfähigkeitssensor nach einem der Ansprüche 1 bis 8, worin der Gürtel eine Gewebe-Innenhülle (13) mit einer solchen Größe aufweist, dass sie um den Körper eines Patienten (12) passt;
wobei die leitende Spule eine erste und eine zweite Spule (14, 16) umfasst, die in beabstandeter Beziehung den Umfang entlang um die Hülle herum angeordnet sind, so dass sie eine in einem Bauchbereich und die andere in einem Brustbereich des Patienten passt, wenn sich die Hülle (13) in Position auf dem Patienten (12) befindet;
wobei der sich selbst abstimmende Oszillator (32) einen ersten und einen zweiten sich selbst abstimmenden Oszillator umfasst, um die erste und die zweite Spule zu Resonanz anzuregen; und
die Impedanzmessschaltung (40, 42) dazu angeordnet ist, einen Messwert der Parallel-Resonanzschaltungen bereitzustellen, welcher die erste bzw. die zweite Spule (14, 16) in Resonanz umfasst.

15. Patienten-Überwachungsgürtel nach Anspruch 14, der eine leitende Außenhülle umfasst, die koaxial um die Außenseite der ersten und der zweiten Spule (14, 16) und die Innenhülle (13) passt.

## Revendications

1. Détecteur de conductivité pour mesurer un matériau de proximité comprenant :
une bobine conductrice (14, 16) réalisant une inductance (L1) produisant un champs magnétique oscillant induisant des courants de Foucault dans le matériau de proximité lorsque la bobine conductrice est excitée avec un courant oscillant ;
un condensateur (C₁) monté en parallèle avec l'inductance pour produire un circuit de résonance parallèle possédant une fréquence de résonance ; et
un oscillateur (32) connecté au circuit de résonance parallèle pour entraîner le circuit de résonance parallèle avec le courant oscillant ; et
un circuit de mesure d'impédance (40, 42) agencé pour fournir une mesure d'une résistance effective du circuit de résonance parallèle ;
où l'oscillateur (32) est un oscillateur d'auto-accord comprenant un amplificateur présentant une sortie et une entrée connectées au circuit de résonance parallèle pour produire une réaction positive amenant le courant oscillant de la sortie à être à une fréquence de résonance du circuit de résonance parallèle couplé au matériau de proximité pour induire des courants de Foucault dans le matériau de proximité ; et
le circuit de mesure d'impédance (40, 42) est agencé pour commander le courant oscillant selon un signal d'erreur pour produire une tension alternative d'amplitude constante dans l'inductance, le signal d'erreur étant la différence entre un signal référence standard (44) et une mesure de l'amplitude de la tension dans l'inductance (11) par quoi le circuit de mesure d'impédance fournit une mesure de la résistance effective du circuit de résonance parallèle couplé au matériau de proximité, qui réflecte la grandeur des courants de Foucault induits dans le matériau de proximité, selon l'amplitude du signal d'erreur.

2. Détecteur de conductivité selon la revendication 1, où l'oscillateur d'auto-accord comporte un amplificateur de commande de gain (32) présentant un signal d'entrée de commande de gain réfléchissant l'amplitude du signal entraînant la bobine ; et
où le circuit de mesure d'impédance obtient la résistance effective du signal de commande de gain.

3. Détecteur de conductivité selon la revendication 2, où le signal de commande de gain est fourni par un redresseur synchrone, redressant un signal d'oscillateur de l'oscillateur d'auto-accord d'une manière synchrone avec le courant oscillant.

4. Conducteur de conductivité selon la revendication 2, où le signal de commande de gain est fourni par un redresseur sous la forme d'un multiplicateur multipliant le signal oscillant de l'oscillateur d'auto-accord par lui-même.

5. Détecteur de conductivité selon la revendication 1, comportant un guide (60) pour positionner le matériau mesuré selon une relation prédéterminée par rapport à la bobine.

6. Détecteur de conductivité selon la revendication 5, où le guide (60) est un tube non-conducteur à travers lequel un liquide (58) peut passer.

7. Détecteur de conductivité selon la revendication 1, où le matériau mesuré est à proximité mais à l'extérieur de la bobine (14, 16).

8. Organe de surveillance d'un patient comportant un détecteur de conductivité selon l'une quelconque des revendications précédentes, dans lequel,
la bobine conductrice (14, 16) est dimensionnée pour s'adapter autour du corps d'un patient (12) ;
l'oscillateur d'auto-accord (32) est agencé pour induire des courants de Foucault dans le tissu du patient ;
le circuit de mesure d'impédance (40, 42) est agencé pour fournir une mesure de la résistance effective du circuit de résonance parallèle telle que réfléchie par la grandeur des courants de Foucault induits dans le tissu du patient (12) ; et
l'organe de surveillance comporte un filtre électronique (50, 52) recevant la mesure d'impédance pour extraire un signal pléthysmographique périodique.

9. Organe de surveillance d'un patient selon la revendication 8, où le filtre (50, 52) est agencé pour fournir un signal pléthysmographique périodique indiquant la respiration par le patient (12).

10. Organe de surveillance d'un patient selon la revendication 8, où le filtre (50, 52) est agencé pour fournir un signal pléthysmographique périodique indiquant les battements de coeur du patient.

11. Organe de surveillance d'un patient selon la revendication 8, comportant de plus un écran conducteur entourant la bobine sur le côté de la bobine au loin du patient.

12. Organe de surveillance d'un patient selon la revendication 8, comportant un circuit d'alarme (58) disposé pour surveiller le signal pléthysmographique périodique pour produire un signal d'alarme lorsqu'une souffrance du patient (12) est indiquée.

13. Organe de surveillance d'un patient selon la revendication 12, comportant deux desdites bobines conductrices (14, 16) dimensionnées pour s'adapter autour du corps du patient (12), une dans une zone abdominale du patient (12), et une dans la zone thoracique du patient où le circuit de mesure d'impédance fournit des mesures séparées de la résistance effective des circuits de résonance parallèles incluant les deux bobines respectives (14, 16) ; et
où le circuit d'alarme (58) compare ces mesures de résistance séparées pour déterminer lorsqu'une souffrance du patient (12) est indiquée.

14. Ceinture de surveillance d'un patient comportant un détecteur de conductivité selon l'une quelconque des revendications 1 à 8, où la ceinture possède un manchon interne en tissu (13) dimensionné pour s'adapter autour du corps d'un patient (12) ;
ladite bobine conductrice comprend une première et une seconde bobine (14, 16) positionnées circonférentiellement autour du manchon selon une relation espacée pour établir une dans une région abdominale et l'autre dans une région thoracique du patient lorsque le manchon (13) est en place sur le patient (12) ;
ledit oscillateur d'auto-accord (32) comprend un premier et un second oscillateur d'auto-accord pour exciter les première et seconde bobines en résonance ; et
ledit circuit de mesure d'impédance (40, 42) est agencé pour fournir une mesure des circuits de résonance parallèles respectivement, incluant les première et seconde bobines (14, 16) à la résonance.

15. Ceinture de surveillance d'un patient selon la revendication 14, comportant un manchon extérieur conducteur s'adaptant coaxialement autour de l'extérieur des première et seconde bobines (14, 16) et du manchon interne (13).
